# EUROPÄISCHE PATENTSCHRIFT

(11) **EP 0 413 802 B1**
(45) Veröffentlichungstag und Bekanntmachung des Hinweises auf die Patenterteilung: **02.11.1994**
(21) Anmeldenummer: 90904315.0
(22) Anmeldetag: 26.02.1990
(51) Int. Cl.: A61B 5/0408

(54) **KÖRPERELEKTRODE UND VERFAHREN ZU IHRER HERSTELLUNG**
BODY ELECTRODE AND PROCESS FOR PRODUCING IT
ELECTRODE CORPORELLE ET PROCEDE POUR SA FABRICATION

(30) Priorität: 27.02.1989 DE 3906071; 27.02.1989 DE 3906074
(43) Veröffentlichungstag der Anmeldung: 27.02.1991
(73) Patentinhaber: SCHMID, Walter, D-89284 Pfaffenhofen (DE)
(72) Erfinder: SCHMID, Walter, D-89284 Pfaffenhofen (DE)
(74) Vertreter: Müller-Boré & Partner Patentanwälte
(86) Internationale Anmeldenummer: EP9000320
(87) Internationale Veröffentlichungsnummer: WO9009758

(56) Entgegenhaltungen:
- EP-A- 0 096 330
- EP-B- 0 085 327
- WO-A-87/05814
- DE-C- 3 136 366
- US-A- 4 066 078
- US-A- 4 852 571

## Beschreibung

Die Erfindung betrifft eine Körperelektrode und ein Verfahren zu ihrer Herstellung.

Derartige Elektroden sind beispielsweise aus den US-Psen 4 554 924, 4 066 078 und 4 362 165 sowie der DE-AS 28 14 061, der DE-OS 35 07 301 und der WO 81/02097 bekannt. Sie werden an der Haut des zu untersuchenden bzw. zu behandelnden Patienten befestigt und dienen dazu, elektrische Signale des Körpers in ein Aufzeichnungsgerät oder umgekehrt derartige Signale aus einer Vorrichtung in den Körper zu übertragen. Beispielsweise werden sie zur Aufzeichnung von Elektrokardiogrammen, als Erdungselektroden bei Operationen oder zur Schmerzlinderung durch Übertragung von elektrischen Impulsen eingesetzt.

Diese Elektroden bestehen im wesentlichen aus einem oder mehreren, in der Regel plattenförmig ausgestalteten galvanisch aktiven Sensor(en), der bzw. die eine Anschlußstelle für eine elektrische Leitung aufweist bzw. aufweisen, die zu einer Aufzeichnungsvorrichtung führt beziehungsweise elektrische Impulse aus einer Vorrichtung in den Körper überträgt, wobei der Sensor an seiner Körperkontaktseite mit einer auf der Haut haftenden Schicht aus einem elektrisch leitenden klebenden elastischen hydrophilen Material oder Materialverbund verbunden ist. Diese Schicht dient dazu, eine innige Haftung der Körperelektrode auf der Haut unter Ausgleich von Körperunebenheiten zu gewährleisten, um die Übertragung von elektrischen Signalen aus dem Körper über den Sensor in ein Anzeigegerät oder ein in einem Gerät erzeugtes Signal über den Sensor in den Körper sicherzustellen. An der dem Körper abgewandten Seite befindet sich eine nichtleitende elastische und nichtklebende Abdeck- oder Stützschicht, welche die Elektrodenplatte und, falls die Elektrodenplatte kleiner ist als die an der Haut klebenden Schicht, diese abdeckt und mit ihr verbunden ist.

Die bekannten Elektroden werden in der Weise hergestellt, daß aus einer breiten Endlosbahn die die Haut kontaktierende klebrige Schicht in der gewünschten Größe und Form ausgestanzt wird, worauf der Sensor auf den erhaltenen Formling aufgesetzt wird und anschließend, falls die die Haut kontaktierende Schicht flächenmäßig größer ist als die Sensorplatte, eine mit der die Haut kontaktierenden Schicht fluchtende Abdeck- und Stützschicht, die ebenfalls durch Ausstanzen erzeugt wird, aufgebracht wird. Dabei treten beim Ausstanzen der die die Haut kontaktierenden klebrigen Schicht erhebliche Probleme auf, da es schwierig ist, die ausgestanzten klebrigen Teile aus dem Stanzwerkzeug zu entnehmen.

Aufgrund der Klebrigkeit der Bahn, aus der die an der Haut haftende Schichtteile ausgestanzt werden, ist der Stanzvorgang mit Schwierigkeiten verbunden, da die ausgestanzten Teile zu einem Kleben an dem Stanzwerkzeug neigen, so daß ein schnelltaktiges kontinuierliches Stanzen schwierig durchzuführen ist.

So zeigt die EP-B-0 085 327 Körperelektrode mit einer an der Haut haftenden Schicht und einer nichtklebenden nichtleitenden Deckschicht sowie einem oder mehreren galvanisch aktiven Sensor(en), der (die) an der Körperkontaktseite mit der an der Haut haftenden Schicht aus einem elektrisch leitenden klebenden elastischen hydrophilen Material verbunden ist (sind), wobei die der Körperkontaktseite abgewandte Seite des Sensors bzw. der Sensoren und der an der Haut haftenden Schicht mit der nichtklebenden nichtleitenden Deckschicht (5) abgedeckt sind, und die an der Haut haftende Schicht aus einem vergießbaren Polymer besteht, welches in eine Form gegossen und anschließend ausgehärtet worden ist. Allerdings ist die Form hinsichtlich Größe und Ausgestaltung nicht so gewählt, daß sie der an der Haut haftenden Schicht entspricht.

Die Erfindung hat sich daher die Aufgabe gestellt, eine Körperelektrode zu schaffen, die unter Vermeidung von Stanzvorgängen hergestellt werden kann.

Sie hat sich weiterhin die Aufgabe gestellt, ein Verfahren zur Herstellung von Körperelektroden zu schaffen, das in störungsfreier sowie einfacher wirtschaftlicher Weise durchführbar ist.

Diese Aufgaben werden durch die Körperelektrode des Patentanspruchs 1 und das Verfahren des Patentanspruchs 7 gelöst.

Demgemäß besteht die erfindungsgemäße Körperelektrode aus einer Haftschicht 4 und einer nichtleitenden Deckschicht 5 sowie einem oder mehreren galvanisch aktiven Sensor(en), der (die) an der Körperkontaktseite mit der an der Haut haftenden Schicht 4 aus einem elektrisch leitenden klebenden elastischen hydrophilen Material verbunden ist (sind), wobei die der Körperkontaktseite abgewandte Seite des Sensors bzw. der Sensoren und der an der Haut haftenden Schicht 4 mit der nichtklebenden nichtleitenden Deckschicht 5 abgedeckt sind, und die an der Haut haftende Schicht 4 und die nichtleitende Deckschicht 5 aus jeweils einem vergießbaren Polymer bestehen, welches jeweils in eine Form gegossen und anschließend ausgehärtet worden ist, wobei Größe und Ausgestaltung der Form so gewählt sind, daß diese der an der Haut haftenden Schicht entspricht, so daß nicht mehr die vorstehend geschilderten Probleme auftreten, die beim Stanzen dieser Schichten, insbesondere der klebrigen, an der Haut haftenden Schicht, auftreten.

Demgemäß besteht das erfindungsgemäße Verfahren darin, daß zur Herstellung der an der Haut haftenden Schicht 4 ein elektrisch leitendes oder leitend gemachtes, klebriges elastisches hydrophiles Polymer und zur Herstellung der nichtleitenden Deckschicht 5 ein nichtklebendes nichtleitendes Polymer in vergießbarer Form eingesetzt wird, wobei nach Eingiessen einer Schicht und hinreichender Verfestigung derselben der Sensor eingelegt und die zweite Schicht aufgegossen und anschließend ausgehärtet wird, wobei Größe und Ausgestaltung der Form so gewählt sind, daß diese der an der Haut haftenden Schicht 4 entspricht.

Da bei dem erfindungsgemäßen Verfahren nicht mehr das Problem des Anklebens der ausgestanzten, an der Haut haftenden Schichtteile besteht, kann es in großtechnischem Maßstabe kontinuierlich störungsfrei mit hohen Taktzeiten durchgeführt werden.

In zweckmäßiger Weise ist das Verhältnis der Fläche der an der Haut haftenden Schicht zu der Fläche des plattenförmigen Sensors bzw. der Sensoren größer als 1 und insbesondere 2 bis 3, d.h. daß in zweckmäßiger Weise ein möglichst kleiner Sensor im Verhältnis zu der die Haut kontaktierenden Schicht verwendet wird. Diese Ausgestaltung ist insbesondere dann zweckmäßig, wenn gemäß einer weiteren erfindungsgemäß bevorzugten Ausführungsform sowohl die an der Haut haftende Schicht als auch die Deckschicht durchsichtig sind, so daß eine Beobachtung der Hautkontaktfläche des behandelten Patienten möglich ist und damit festgestellt werden kann, ob, insbesondere beim längeren Anlegen der Elektrode, Hautreizungen auftreten.

Die an der Haut haftende Schicht besteht in zweckmäßiger Weise aus einem leitenden natürlichen und/oder synthetischen Material in Form eines polymeren Materials, das beispielsweise ein Elastomeres oder ein Kollagen sein kann. Ferner kann diese Schicht aus einem nichtleitenden natürlichen oder synthetischen Material bestehen, das dann zur Erzeugung der Leitfähigkeit einen Elektrolyten enthält Ferner kann es Zweckmäßig sein, wenn die an der Haut haftende Schicht ein klebrigmachendes Mittel, einen Weichmacher und/oder hygroskopisches Mittel enthält, da auf diese Weise die Hafteigenschaften, die elastischen Eigenschaften sowie die Hygroskopizität maßgeschneidert werden können.

Die Deckschicht besteht vorzugsweise aus einem natürlichen oder synthetischen nichtleitenden Material in Form eines Polymeren, wobei Elastomere oder Kollagene bevorzugt werden.

Die an der Haut haftende Schicht und die Deckschicht können aus das selbe Material aufweisen, wobei natürlich die an der Haut haftende Schicht durch Zusatz eines Elektrolyten leitfähig und darüber hinaus klebrig gemacht werden muß. Die Klebrigkeit kann durch Zusatz eines klebrigmachenden Mittels oder, falls vernetzbare Polymere zur Herstellung der beiden Schichten eingesetzt werden, in der Weise erzielt werden, daß die die Haut kontaktierende Schicht weniger stark vernetzt wird als die Deckschicht, falls das Polymere in weniger stark vernetztem Zustand eine ausreichende Klebrigkeit besitzt.

Gemäß einer weiteren bevorzugten Ausführungsform kann die erfindungsgemäße Körperelektrode, falls die an der Haut haftende Schicht und die Deckschicht durchsichtig sind, eine dazwischen liegende durchsichtige Schicht aufweisen, die mit Zeichen, beispielsweise Beschriftungen oder graphischen Darstellungen versehen ist, um beispielsweise auf diese Weise die Elektrode herkunftsmäßig zu identifizieren.

Die erfindungsgemäßen Körperelektroden enthalten 1 oder mehrere Sensor(en) und können vorzugsweise kreisförmig oder quadratisch ausgestaltet sein, wobei sie jedoch auch in beliebigen anderen Formen gefertigt werden können.

Als Sensoren für die erfindungsgemäße Körperelektrode kommen alle bekannten Sensoren in Frage, beispielsweise diejenigen, die in der DE-OS 24 59 627, in der US-PS 4 554 924, in der US-PS 4 066 078, in der DE-AS 28 14 061 sowie in der DE-OS 29 35 238 beschrieben werden. Als besonders zweckmäßig hat sich ein plattenförmiger, mit einer druckknopfartigen Anschlußstelle für eine elektrische Leitung ausgestatteter Sensor erwiesen, der aus Kunststoff besteht, welcher mit einem leitenden, galvanisch aktiven Material überzogen ist, beispielsweise einer Silber/Silberchlorid-Schicht. Vorzugsweise können die Sensoren auch aus Polymeren bestehen, die ein reversibles Redoxsystem darstellen. Derartige Materialien sind bekannt.

Im allgemeinen wird in der erfindungsgemäßen Körperelektrode zwar ein plattenförmiger Sensor, der meistens rund ist, verwendet, es ist jedoch auch möglich, als Sensor den bzw. die Leiter der elektrischen Leitung bzw. Leitungen, mit der bzw. mit denen die Signalübertragung in die Aufzeichnungsvorrichtung erfolgt, zu verwenden. Vorzugsweise wird bei dieser besonders preiswerten Ausführungsform ein elektrisches Leitungskabel mit einer Silberlitze verwendet, die mit einer Silberchloridschicht überzogen ist.

Ferner können als galvanisch aktive Sensoren beispielsweise aus Kunststoffen bestehende Mikrokügelchen, die mit Zink oder Silber überzogen sind, verwendet werden, beispielsweise die unter dem Warenzeichen Metalite vertriebenen Materialien, die in einer entsprechenden Anzahl zwischen der die Haut kontaktierenden Schicht und der Deckschicht positioniert werden und mit einem zu einer Aufzeichnungsvorrichtung führenden elektrischen Leiter verbunden sind.

Die Sensoren und/oder die elektrischen Leitungen können auch aus Schichten bestehen, die auf eine Folie, beispielsweise durch Siebdrucken aufgebracht worden sind.

Falls in den erfindungsgemäßen Elektroden ein plattenförmiger Sensor verwendet wird, weist dieser vorzugsweise in seinem Plattenteil Ausnehmungen, insbesondere Löcher, auf, welche eine bessere Verankerung der die Haut kontaktierende Schicht bzw. der Deckschicht gewährleisten.

Es ist auch möglich, von der erfindungsgemäßen Körperelektrode aus dem Körper aufgenommene elektrische Signale drahtlos in eine Aufzeichnungsvorrichtung zu übertragen. Zu diesem Zweck kann die Elektrode mit einem integrierten Schaltkreis mit geringer Stromaufnahme als Sender, der mit einer Stromquelle, beispielsweise einer Knopfzelle, betrieben wird, ausgestattet werden.

Soll die erfindungsgemäße Elektrode beispielsweise auf dem Brustkorb angesetzt werden, dann ist es zweckmäßig, wenn sie in der Längsrichtung elastisch ist. Für diesen Fall ist eine Ausführungsform einer erfindungsgemäßen Körperelektrode vorgesehen, die aus mehreren Elektrodeneinheiten besteht, welche durch eine Zieharmonika-artig gefaltete Folie verbunden sind, die ein Ausdehnen der Elektrode in Längsrichung, beispielsweise beim Einatmen und der damit verbundenen Vergrößerung des Brustkorbs, gestattet.

Die Erfindung wird durch die beigefügten Figuren 1 bis 7 naher erläutert, wobei in diesen Figuren gleiche Teile durch gleiche Bezugszeichen gekennzeichnet sind.

Es zeigen:
- Fig. 1: einen Senkrechtschnitt durch eine Ausführungsform einer erfindungsgemäßen Elektrode mit einem Sensor,
- Fig. 2: eine Draufsicht auf eine erfindungsgemäße Körperelektrode, die mit drei Sensoren versehen ist,
- Fig. 3: eine Draufsicht auf eine andere Ausführungsform einer derartigen Elektrode,
- Fig. 4: eine Draufsicht auf eine Ausführungsform auf einer derartigen Elektrode, die mit einem Sender ausgestattet ist,
- Fig. 5: eine Draufsicht auf eine als Stimulationselektrode ausgebildete erfindungsgemäße Elektrode,
- Fig. 6: einen Senkrechtschnitt durch eine in Längsrichtung ausdehnbare erfindungsgemäße Elektrode und
- Fig. 7: eine Draufsicht auf eine kreisförmige erfindungsgemäße Elektrode mit einem Sensor, dessen Plattenteil mit Löchern versehen ist..

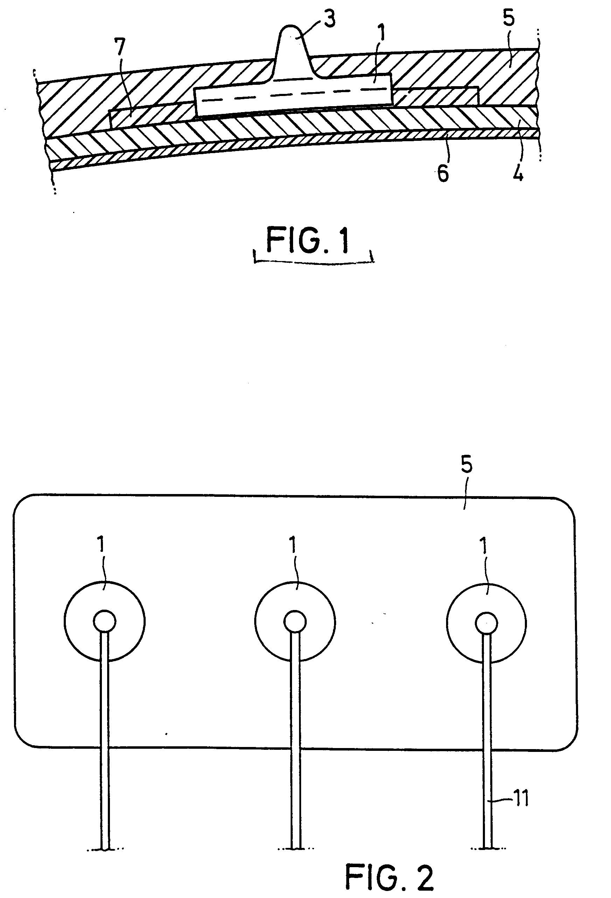

Die durch die Fig. 1 wiedergegebene Ausführungsform einer erfindungsgemäßen Körperelektrode besteht aus einem plattenförmigen runden Sensor 1 mit einer Anschlußstelle für eine elektrische Leitung, die zu einer Aufzeichungsvorrichtung führt. Der Sensor kann aus einem solchen bestehen, wie er in den weiter oben genannten Literaturstellen beschrieben wird. An seiner dem Körper zugewandten Seite ist der Sensor mit einer an der Haut haftenden Schicht 4 versehen, die aus jedem beliebigen bekannten elastischen, hautfreundlichen, nichttoxischen, hydrophilen und an der Haut klebenden natürlichen oder synthetischen Material bestehen kann. Voraussetzung für dieses Material ist, daß es in irgendeiner Form vergießbar ist. Vorzugsweise besteht das in Frage kommende Material aus einem synthetischen, vorzugsweise elastomeren Polymeren oder aus einer Polymermischung und/oder aus einem Kollagen, wobei dieses Material entweder selbstleitend ist oder durch Zusatz eines Elektrolyten leitend gemacht worden ist. Vorzugsweise werden zur Herstellung der Schicht 4 synthetische organische Polymere oder Polymermischungen verwendet, die entweder selbstleitend sind oder durch Zusatz eines Elektrolyten leitend gemacht worden sind. Diese Polymeren müssen in Form einer Lösung, einer Schmelze oder eines entsprechend niedrigviskosen Vorpolymeren vergießbar sein. Polymere, welche diesen Anforderungen genügen, stehen in reicher Auswahl zur Verfügung. Als leitende Polymere kommen beispielsweise die in der US-PS 4 066 078 beschriebenen Polymeren aus 2-Acrylamido-2-methylpropansulfonsäure, ihren Salzen, Copolymeren der genannten Säure, Copolymeren der Salze der Säure sowie Mischungen davon in Mischung mit Wasser, Alkoholen und Mischungen davon, die in der DE-OS 29 35 238 beschriebenen Polymeren mit mindestens 5 Mol-% Monomereinheiten, die ein Salz einer Carbonsäure enthalten, sowie die leitenden polymeren Massen in Frage, welche aus einem hydrophilen vernetzten Polymeren und einem hydrophilen nichtvernetzten Polymeren bestehen und in der EP 85 327 beschrieben werden.

Von den Polymeren, die als solche nicht leitfähig sind und durch Zusatz eines Elektrolyten leitfähig gemacht werden, seien Polyvinylalkohole, Polyvinylacetate, Polyvinylpropionate, Polyvinylether, wie sie beispielsweise in "Adhäsion" 5/81, Seiten 208 bis 213 beschrieben werden, sowie Polyacrylate und Polymethacrylate erwähnt, beispielsweise diejenigen, die in der DE-OS 31 36 366, der US-PS 4 554 924 und in der DE-AS 28 14 061 beschrieben werden.

Als besonders geeignet erwiesen haben sich die von der BASF unter dem Warenzeichen "LAROMER" in den Handel gebrachten ungesättigten Acrylatharztypen erwiesen, in ganz besonders bevorzugter Weise das Harz, das unter dem Warenzeichen "LAROMER EA-8812" vertrieben wird.

Zur Erhöhung bzw. zur Steuerung der Klebrigkeit können diese Polymeren zusätzlich eines oder mehrere klebrigmachende Mittel, wie die für diesen Zweck bekannten Glycidetheracrylate und deren Derivate, enthalten. Außerdem können sie Weichmacher und/oder hygroskopische Mittel enthalten, wie Polyole, wie Diole und Triole, beispielsweise Hexantriole, Polyethylenglykol etc. In zweckmäßiger Weise enthalten sie noch einen Zusatz an Bakteriostatika sowie Geruchsbinder, wie Eukalyptol.

Wie bereits erwähnt, werden die eingesetzten Polymeren zur Herstellung der die Haut kontaktierenden Schicht 4 in einer Gießform in einem gießfähigen Zustand vergossen, und zwar entweder in Form einer Lösung, einer Schmelze oder eines durch Vernetzung härtbaren Vorpolymeren. Die zuletztgenannte Ausführungsform wird bevorzugt, wobei die Aushärtung vorzugsweise durch Bestrahlung mit UV-Licht und/oder Wärme unter Einsatz bekannter Photoinitiatoren und/oder Härtungsbeschleuniger wie unter Einsatz von Perverbindungen, wie Peroxiden, und/oder Ketonhärtungsbeschleunigern, erfolgt, beispielsweise unter Verwendung von 2-Hydroxy-2-methyl-1-phenyl-propan-1-on, das ein wirksamer Photoinitiator für acrylierte Epoxide, acrylierte Polyurethane, acrylierte Polyether und acrylierte ungesättigte Polyester oder Mischungen davon ist.

Durch Variation der Art und Menge des eingesetzten leitenden bzw. durch Elektrolytzusatz leitend gemachten Polymeren, des gegebenenfalls eingesetzten klebrigmachenden Mittels, Weichmachers und/oder hygroskopischen Mittels sowie des Vernetzungs- bzw. Härtungsgrades lassen sich die Klebe-, Leitfähigkeits- und Elastizitätseigenschaften der die Haut kontaktierenden Schicht 4 maßschneidern.

Als Elektrolyten, die dazu verwendet werden, nichtleitende Polymere zur Herstellung der Schicht 4 leitfähig zu machen, werden vorzugsweise Alkalihalogenide, wie Kaliumchlorid, verwendet.

Das Material für die Deckschicht 5 kann jedes bekannte natürliche und/oder synthetische hydrophile Material sein, das ebenfalls in Form einer Lösung, einer Schmelze oder eines vernetz- oder aushärtbaren Vorpolymeren vergießbar ist und sich beim Verfestigen mit dem Material der Schicht 4 verbindet. Das Material für die Deckschicht 5 darf natürlich nicht leitend sein und auch keine leitend machenden Zusätze enthalten und kann ebenso wie das Material der hautkontaktierenden Schicht durchsichtig sein. Von den vielen bekannten, den genannten Kriterien genügenden Materialien seien Polymere, insbesondere elastomere Polymere, Gelatine, insbesondere mit einer Gallertfestigkeit von 60 bis 260 Bloom Ossein und einer Shore-Härte der gegossenen Gelatine zwischen 40 und 90 Shore, sowie insbesondere die auch zur Herstellung der Schicht 4 verwendeten Polyacrylate erwähnt, die jedoch soweit vernetzt bzw. gehärtet werden, daß sie nicht mehr klebrig sind. Vorzugsweise werden daher zur Herstellung der Deckschicht 5 bevorzugt die selben Polyacrylatvorpolymeren eingesetzt, wie sie zur Herstellung der hautkontaktierenden Schicht verwendet werden, wobei sie jedoch in einem stärkeren Ausmaße vernetzt bzw. gehärtet werden, daß sie nicht mehr klebrig sind, erwähnt. Auf diese Weise entsteht ein besonders fester Verbund aus dem Polymeren der Schicht 4 und dem Polymeren der Schicht 5.

In der in Fig. 1 gezeigten Ausführungsform befindet sich zwischen der die Haut kontaktierenden Schicht 4 und der Deckschicht 5 eine mit Zeichen versehene Zwischenschicht, die ebenso wie die Deckschicht und die die Haut kontaktierende Schicht aus einem durchsichtigen Material besteht, damit man bei einer Betrachtung der Elektrode die auf dieser Schicht 7 aufgebrachten Zeichen erkennen kann. Diese Schicht besteht ebenfalls aus einem vergießbaren natürlichen oder synthetischen Material, wobei die selben Materialien verwendet werden können, wie sie zur Herstellung der Schichten 4 und 5 verwendet werden. Die Schicht 7 muß jedoch nicht leitfähig sein.

Ferner können Zeichen auf der dem Körper abgewandten Oberseite der Schicht 5 aufgebracht sein, beispielsweise durch Aufdrucken, Aufkleben oder Aufmalen, wobei jedoch in zweckmäßiger Weise etwaige Zeichen in erhabener Form auf der Oberseite der Deckschicht 5 angebracht sind, die durch Vergießen der Deckschicht in einer Form mit entsprechenden Vertiefungen erhalten worden sind. In diesem Falle wird nicht, wie weiter unten ausgeführt, die Körperelektrode in der Weise hergestellt, dar zuerst die hautkontaktierende Schicht vergossen, der Sensor aufgesetzt und dann die Deckschicht vergossen wird, sondern es wird in der umgekehrten Weise verfahren.

Die an der Haut haftende Schicht ist mit einer abziehbaren Schutzschicht 6 versehen, die aus jedem beliebigen, sich von der an der Haut haftenden Schicht lösbaren Material, das vorzugsweise hydrophob und/oder mit einem Trennüberzug, beispielsweise einem Silikonüberzug versehen ist, bestehen kann. In besonders zweckmäßiger Weise besteht die Schutzschicht 6 aus der Form, in welcher die Körperelektrode durch Vergießen der Schichten 4 und 5, sowie gegebenenfalls 7 hergestellt worden ist, d.h., daß die Körperelektrode nach dem Vergießen nicht aus der Form entnommen wird, sondern mit dieser Gießform als Einheit in den Handel gebracht wird, wobei beim Gebrauch der Elektrode die Gießform abgezogen wird. Vorzugsweise weist die Deckschicht 5 eine (nicht gezeigte) Lasche auf, um das Herausziehen aus der Form zu erleichtern.

Die durch die Fig. 2 wiedergegebene erfindungsgemäße Körperelektrode ist rechteckig ausgestaltet und enthält drei kreisförmige Sensoren, wobei jedoch in diesem Falle die elektrischen Leiter, mit welchen diese Sensoren in einer Anzeigevorrichtung verbunden werden können, nicht wie im Falle der Ausführungsform der Fig. 1 an der dort gezeigten druckknopfartigen Anschlußstelle 3 mit dem Sensor verbunden sind, sondern an der Sensoren ohne druckknopfartige Anschlußvorrichtung befestigt sind und in der horizontalen Ebene der Körperelektrode in der Deckschicht 5 eingebettet sind, an deren seitlichem Rand sie austreten. Diese elektrischen Leiter 11 können aus üblichen elektrischen Anschlußkabeln bestehen, vorzugsweise bestehen sie jedoch aus Carbonfasern und/oder leitenden Kunststoffen.

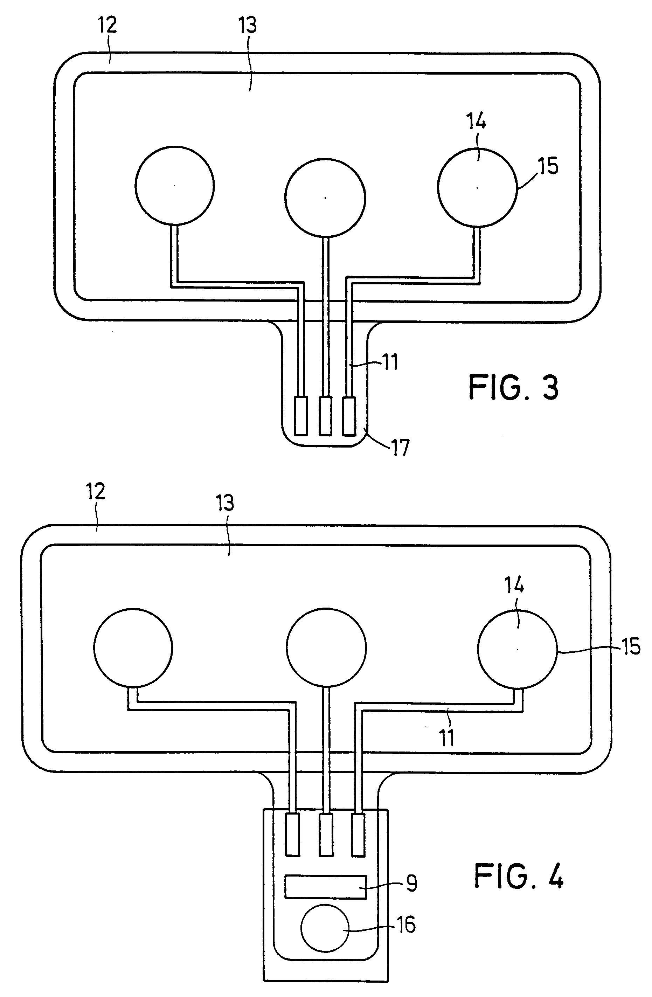

Die Fig. 3 zeigt ebenfalls eine rechteckige erfindungsgemäße Körperelektrode mit drei Sensoren, wobei die mit diesen Sensoren verbundenen elektrischen Leitungen 11 in einer Anschlußzunge 17 münden, von welcher aus ein Anschluß an eine Aufzeichnungsvorrichtung möglich ist. Im Falle dieser Ausführungsform sind in der vergossenen Deckschicht 12 runde Aussparungen 14 vorgesehen, welche mit einem leitenden klebenden Material, wie es zur Herstellung der an der Haut haftenden Schicht verwendet wird, gefüllt sind. Auf diesem Gebilde liegt eine an den Stellen 15 mit einer galvanisch aktiven Schicht beschichtete Folie 13, welche ebenfalls durch Beschichten aufgebrachte Anschlußleitungen 11 aufweist auf. Das Beschichten dieser Folie 13 mit der galvanisch aktiven Schicht bzw. mit den elektrischen Leitungen kann beispielsweise in Siebdruckverfahren erfolgen.

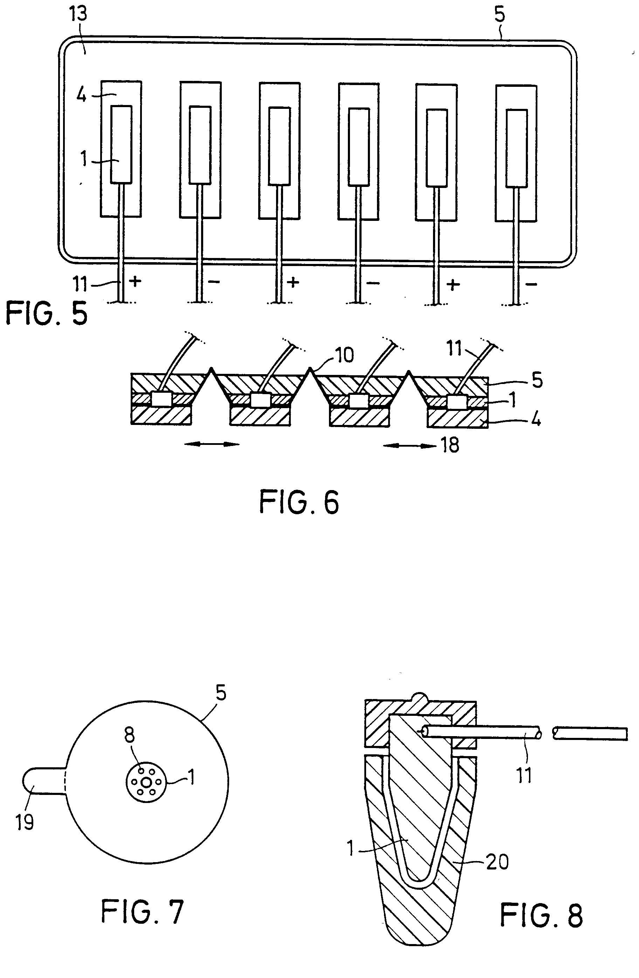

Die Fig. 4 zeigt eine ähnliche Ausführungsform wie die Fig. 3, wobei jedoch in diesem Falle die elektrischen Leitungen 11 mit einem durch eine Knopfzelle 16 betriebenen Sender 9 in Verbindung stehen, der drahtlos die aus dem Körper übertragenen Signale in eine Aufzeichnungsvorrichtung weiterleitet.

Die Fig. 5 zeigt eine erfindungsgemäße Elektrode, die als Stimulationselektrode mit nach Bedarf wählbarer Polarität verwendbar ist. Diese Elektrode besteht aus einer vergossenen Deckschicht 5 mit Ausnehmungen, in welchen das Material der an der Haut haftenden Schicht 4 vergossen ist, wobei auf der Folie 13 entsprechend der durch Fig. 3 gezeigten Ausführungsform galvanisch aktive Sensoren 1 mit den entsprechenden elektrischen Leitungen 11 aufgebracht sind, die mit der auf der Haut haftenden Schicht 4 in Kontakt stehen.

Mit einer derartigen Elektrode werden in einem Gerät erzeugte Signale übertragen, beispielsweise zur Schmerzlinderung.

Die Fig. 6 zeigt eine aus mehreren Einzelelektroden bestehende in der Längsrichtung 18 dehnbare Elektrode, die aus vier Einzelelektroden besteht, welche durch eine Zieharmonika-förmig gefaltete Folie 10 an den Seiten ihrer Deckschicht 5 verbunden sind. Die verwendete Folie besteht vorzugsweise aus einem ohne Rißbildung knickbaren, insbesondere elastomeren Kunststoff. Die vier Sensoren dieser Elektrode sind mit Leitungen 11 mit einem Anzeigegerät verbindbar. Bei dieser Ausführungsform sowie bei den durch die Fig. 1 gezeigten Ausführungform kann der Sensor 1 aus einem Streifen aus einem galvanisch aktiven Metall oder aus einem Kunststoff, der mit einem galvanisch aktiven Metall überzogen ist, bestehen, wobei dieser Streifen in abgewinkelter Form mittig durch die Deckschicht 5 herausragt und mit einer elektrischen Leitung mit einem Anzeigegerät verbunden werden kann.

Die Fig. 7 zeigt in Draufsicht eine Körpereleketrode, die im Prinzip derjenigen der Fig. 1 entspricht, wobei die Platte der kreisförmigen Elektrode mit Löchern 8 versehen ist, um eine bessere Verankerung sowohl in der (nicht gezeigten) die Haut kontaktierenden Schicht und der Deckschicht 5 zu gewährleisten, da beim Aufsetzen der Elektrodenplatte auf die frisch vergossene an der Haut haftende Schicht sowie beim Vergießen der Deckschicht 5 die Löcher von den jeweiligen Materialien durchdrungen werden, wodurch eine gute Verankerung bewirkt wird. Diese Elektrode besitzt an ihrer Deckschichtseite eine Lasche 19, um das Herausnehmen aus der Gießform zu erleichtern.

Die Dicke der die Haut kontaktierende Schicht 4 bei den vorstehend beschriebenen Ausführungsformen flach ausgebildeter Körperelektroden liegt vorzugsweise zwischen 800 µm und 2500 µm und in ganz besonders bevorzugter Weise zwischen 1000 µm und 2000 µm, während die Dicke der Abdeckschicht 5 vorzugsweise zwischen 500 µm und 2500 µm und insbesondere zwischen 1000 und 2000 µm variiert. Kreisförmige Elektroden der durch die Fig. 1 und 7 gezeigten Ausführungsformen besitzen vorzugsweise Durchmesser zwischen 20 mm und 60 mm.

Die Dicke der Elektrodenplatte von in diesen Elektroden eingesetzten plattenförmigen Elektroden schwankt vorzugsweise zwischen 50 µm und 1500 µm, insbesondere zwischen 80 µm und 1000 µm.

Eine weitere nicht unter die Ansprüche fallenden Ausführungsform einer Elektrode besteht aus einem vorzugsweise am Kopf anzubringenden und zur EEG-Abnahme zu verwendenden Kontakthütchen, wie es durch die Fig. 8 gezeigt wird.

Dieses Kontakthütchen besteht aus einem durch Vergießen hergestellten konusförmigen und am sich erweiterenden Ende offenen Kontaktkörper 20 aus einem nichtklebenden leitenden Material, in welchem ein Sensor 1 mit einer elektrischen Anschlußleitung 11 eingepaßt ist und durch eine Klemmkraft den Kontaktkörper 20 festhält. Dieses Kontakthütchen wird unter Verwendung eines Klebebandes oder dergleichen an den zu messenden Körperteilen, vorzugsweise am Kopf, befestigt.

Zum Schutz des Kontaktkörpers 20 kann dieser in der Form, in welcher er vergossen worden ist, als Schutzschicht konfektioniert und in den Handel gebracht werden.

Zur Herstellung der die Haut kontaktierenden Schicht werden die obengenannten Polymere in einer Gießform in einem gließfähigen Zustand vergossen, und zwar entweder in Form einer Lösung, einer Schmelze oder eines durch Vernetzung härtbaren Vorpolymeren.

Das zur Herstellung der Stütz- oder Abdeckschicht verwendete Material kann jedes natürliche und/oder synthetische hydrophile Material insbesondere polymeres Material, sein, das ebenfalls in Form einer Lösung, einer Schmelze oder eines vernetz- oder aushärtbaren Vorpolymeren vergießbar ist und sich beim Verfertigen mit dem Material der hautkontaktierenden Schicht verbindet. Das Material für die Deckschicht darf natürlich nicht leitend sein und auch keine leitend machenden Zusätze enthalten und kann ebenso wie das Material der hautkontaktierenden Schicht durchsichtig sein. Die in dem erfindungsgemäßen Verfahren einzusetzenden Polymere sind eingangs erwähnt worden.

Gemäß einer bevorzugten Ausführungsform des erfindungsgemäßen Verfahrens kann, falls entweder die an der Haut haftende Schicht und/oder die Abdeck- oder Stützschicht, insbesondere die letztere, aus einem durchsichtigen Material bestehen, zwischen diese beiden Schichten eine ebenfalls vorzugsweise durchsichtige zeichentragende Schicht eingebracht werden, wobei die Zeichen beispielsweise aus Beschriftungen oder graphischen Darstellungen bestehen, um die Elektrode herkunfts- oder verwendungsmäßig zu identifizieren.

Das gegebenenfalls zur Herstellung der mit Zeichen versehenen Zwischenschicht eingesetzte Material kann ebenfalls aus einem vergießbaren natürlichen oder synthetischen Material bestehen, wobei dieselben Materialien verwendet werden können, wie sie zur Herstellung der die Haut kontaktierenden Schichten und der Deckschichten verwendet werden. Die Zwischenschicht muß jedoch nicht leitfähig sein.

Die zur Durchführung des erfindungsgemäßen Verfahrens eingesetzten Sensoren sind im wesentlichen bekannt und werden ebenfalls in den obengenannten Veröffentlichungen beschrieben.

Eine Identifizierung der erfindungsgemäß hergestellten Elektroden ist auch in der Weise möglich, daß in die Gießform, beispielsweise mit Hilfe eines Prägestempels, Zeichen oder Beschriftungen (beispielsweise Firmenherkunftsangaben oder Typenbezeichnungen) in Spiegelschrift eingeprägt werden, worauf zuerst die Abdeck- oder Stützschicht vergossen wird. Die auf diese Weise hergestellten Elektroden sind dann in ihrer Oberfläche auf der Abdeck- oder Stützschicht mit den erhaben ausgeformten Zeichen versehen.

Nachfolgend wird das erfindungsgemäße Verfahren unter Bezugnahme auf synthetische elastomere polymere Materialien, die zur Herstellung der an der Haut haftendenden Schicht und der Abdeck- oder Stützschicht einer Elektrode, die aus einer die Haut kontaktierenden Schicht, einem plattenförmigen Sensor mit einer Anschlußstelle für eine elektrische Leitung, wobei die Fläche der Sensorplatte kleiner ist als die Fläche der die Haut kontaktierenden Schicht, und einer die dem Körper abgewandte Seite der die Haut kontaktierenden Schicht und der Sensorplatte abdeckenden Abdeck- oder Stützschicht besteht, eingesetzt werden, beschrieben.

Das erfindungsgemäße Verfahren kann, wie bereits erwähnt, in der Weise durchgeführt werden, daß eine Schmelze aus dem zur Bildung an der Haut haftenden Schicht verwendeten Polymeren erzeugt wird, diese Schmelze kontinuierlich mit einem Dosiergerät in eine Form, die der Größe und Ausgestaltung der die an der Haut haftenden Schicht entspricht, in einer solchen Menge eingebracht wird, daß die gewünschte Schichtdicke erzielt wird. Nachdem die Schmelze zumindest, soweit abgekühlt beziehungsweise sich so weit verfestigt hat, daß beim Aufsetzen des Sensors dieser nicht allzu tief in die an der Haut haftende Schicht eindringt, wird dieser auf die erzeugte Schicht aufgesetzt, worauf aus einer nachgeschalteten Dosiervorrichtung die Abedeck- oder Stützschicht in der gewünschten Dicke aufgegossen wird.

Es ist ferner möglich, eine Lösung eines Polymeren in einem geeigneten Lösungsmittel zum Vergießen der an der Haut haftenden Schicht zu verwenden. In diesem Falle läßt man nach dem Vergießen der Schicht das Lösungsmittel, gegebenenfalls beschleunigt durch Erwärmen, zumindest soweit verdampfen, daß der aufgesetzte Sensor nicht unerwünscht tief in die gebildete Schicht eindringt.

Diese Vernetzung bzw. Härtung kann in bekannter Weise durch Wärme, Licht, ionisierende Strahlung oder unter Einsatz von Katalysatoren oder Initiatoren bzw. Härtungs- und Polymerisationsbeschleunigern durchgeführt werden, wobei auch Kombinationen dieser Maßnahmen angewendet werden können, wobei sich die jeweiligen Maßnahmen auch nach der Art des verwendeten polymeren Materials richten.

Katalysatoren, Initiatoren oder Härtungs- bzw. Beschleunigungspromotoren werden in zweckmäßiger Weise der Gießlösung unmittelbar vor dem Vergießen zugesetzt.

Erfindungsgemäß kommt ferner eine Methode in Frage, nach welcher eine vernetz- oder härtbare Vorstufe des verwendeten Polymeren in gießfähigem Zustand vergossen wird. Nach dem Vergießen wird in zweckmäßiger Weise vor dem Aufsetzen des Sensor zumindest soweit vernetzt beziehungsweise gehärtet, daß die vergossene Schicht nicht mehr so weich ist, daß der Sensor zu tief in sie eindringen kann.

Nach einer der drei genannten Methoden wird auch die Abdeck- oder Stützschicht vergossen, wobei jedoch die jeweils eingehaltene Methode nicht mit der Methode identisch sein muß, nach welcher die auf der Haut haftende Schicht vergossen worden ist.

In besonders zweckmäßiger Weise werden jedoch sowohl zur Herstellung der an der Haut haftenden Schicht als auch der Abdeck- oder Stützschicht dieselben vernetz- beziehungsweise härtbaren Polymervorstufen verwendet, wobei die Abdeck- oder Stützschicht, die nicht mehr klebrig sein darf, durch eine oder mehrere der genannten Maßnahmen stärker vernetzt wird als die auf der Haut haftende Schicht, die klebrig bleiben muß. Die Vernetzung kann in der beschriebenen Weise durchgeführt werden.

Die zur Durchführung des erfindungsgemäßen Verfahrens verwendete Gießform wird vorzugsweise in der Weise hergestellt, daß sie aus einer Folie in der gewünschten Form und Tiefe tiefgezogen wird. Zur großtechnischen Herstellung werden daher aus einer Bahn aus einem Material, das sich nicht mit dem Material der an der Haut haftenden Schicht verbindet, sondern sich vielmehr leicht und ohne Hinterlassung von Rückständen von dieser abziehen läßt, durch Tiefziehen entsprechende nebeneinander und hintereinander liegende Formen hergestellt, die dann aufeinanderfolgend unter Dosierungsvorrichtungen zum Zuführen des vergießbaren Materials für die Abdeck- oder Stützschicht in dem entsprechenden Takt, in welchem die genannten Forrichtungen arbeiten, geführt werden. Den jeweiligen Dosiervorrichtungen können Bestrahlungs- und/oder Erhitzungsvorrichtungen nachgeschaltet sein, mit deren Hilfe die erwähnten Vernetzungs- beziehungsweise Aushärtungsreaktionen durchgeführt beziehungsweise beschleunigt werden können.

Vorzugsweise bestehen die Materialien für die Bahn, aus welcher die Formen zum Vergießen der erfindungsgemäßen Körperelektrode tiefgezogen werden, aus einem hydrophoben Polymeren oder aus einem Polymeren, das einen Überzug aus einem Antihaft- bzw. Trennmittel aufweist, beispielsweise einem hydrophoben Silikon. Es kommen auch Metallfolien, beispielsweise Aluminiumfolien, in Frage.

In zweckmäßiger Weise kann die Form, in welcher die erfindungsgemäße Körperelektrode durch Vergießen hergestellt worden ist, als Schutzschicht verwendet werden, um die an der Haut haftende Schicht gegenüber einer Beschädigung oder einem Austrocknen zu schützen. In diesem Falle wird die Elektrode als Einheit mit der zu ihrer Herstellung verwendeten Gießform in den Handel gebracht.

Eine Variante des erfindungsgemäßen Verfahren besteht darin, daß in einer Gießform die auf der Haut haftende Schicht vergossen und ausgehärtet wird, ein Sensor mit elektrischer Anschlußmöglichkeit eingelegt und anschließend die Abdeck- oder Stützschicht unter Einbettung des Sensors vergossen wird.

Eine andere Variante des erfindungemäßen Verfahrens besteht darin, in eine Gießform eine Folie einzulegen, auf welcher ganz oder teilweise eine galvanisch aktive Schicht, gegebenenfalls in bestimmten Konfigurationen, beispielsweise nach einem Siebdruckverfahren aufgebracht worden ist.

Aus dieser Folie wird in Kontakt mit der galvanisch aktiven Schicht die an der Haut haftende Schicht vergossen und nach dem Verfestigen mit einer Abdeckfolie überzogen.

Im Falle der Herstellung einer derartigen Körperelektrode kann die Verbindung der galvanisch aktiven Schicht mit einem Aufzeichnungsgerät in der Weise erfolgen, daß entweder vor dem Vergießen der die Haut kontaktierenden Schicht seitlich oder nach oben aus der Elektrode herausragende Anschlußleitungen in die Form eingelegt werden, die dann mit Leitungen zu den Aufzeichnungsgeräten verbunden werden können.

Die Leitungen können jedoch auch ebenso wie die galvanisch aktive Schicht in Form einer Schicht auf die in die Gießform einzulegende Folie aufgebracht werden, vorzugsweise in einer Dicke von 30 µm bis 200 µm, wobei sie in zweckmäßiger Weise in einer Zunge an der Elektrode münden, von wo aus sie dann mit einer üblichen elektrischen Leitung mit einem Aufzeichnungsgerät verbunden werden.

Abschließend sei noch auf einige bevorgzugte Ausführungsformen hinsichtlich Ausbildung der Elektroden und deren Herstellung hingewiesen:
Die an der Haut haftende Schicht 4 und die Deckschicht 5 sind durchsichtig, die Elektrode selbst ist kreisförmig oder quadratisch, die an der Haut haftende Schicht 4 ist aus einem leitenden natürlich und/oder synthetischen Polymer, insbesondere aus einem nichtleitenden Polymer, das einen Elektrolyten enthält. Die Deckschicht 5 ist aus einem nichtleitenden natürlichen oder synthetischen Polymer, die an der Haut haftende Schicht 4 weist einen abtrennbaren Schutzüberzug 6 auf, wobei der Schutzüberzug aus der Form bestehen kann, in welcher die Elektrode durch Vergießen hergestellt worden ist. Die an der Haut haftende Schicht und die Deckschicht 5 sind durchsichtig, und zwischen diesen beiden Schichten liegt eine mit Zeichen versehene, durchsichtige Schicht 7. Der Sensor bzw. die Sensoren 1 weisen eine Anschlußstelle 3 für eine elektrische Leitung 11 auf, wobei der Sensor oder die Sensoren 1 selbst die Leiter der elektrischen Leitungen 11 sein können. Die Sensoren 1 sind plattenförmig und weisen Ausnehmungen 8 auf, der Sensor bzw. die Sensoren 1 können mit einem Sender 9 verbunden sein. Die Körperelektrode kann auch mehrere Sensoren 1 enthalten, und schließlich kann die Körperelektrode in Längsrichtung dehnbar sein.

Das folgende Beispiel erläutert die Erfindung:
Eine Mischung aus 100 g 1-Vinyl-2-pyrrolidon, 10 g Wasserstoffperoxid, (3 gew.-%ige Lösung in Wasser), 7 g 4-(Hydroxyethoxy)phenyl-(2-hydroxy-2-propyl)-keton, 5 g Polyetheracrylat, 15 g Polyvinylpyrrolidon, 100 g Propantriol, 20 g destilliertes Wasser, 4 g Triethanolamin und 5 g Ammoniumhydroxid (10 % in Wasser) wurde hergestellt. Zu dieser Mischung wurden 40 g einer Lösung aus 30 g Kaliumchlorid und 5 g Natriumtetraborat in 100 g Wasser gegeben.

Aus der erhaltenen Mischung wurden in aus einer Folie aus Polyethylen mit einem Silikonüberzug tiefgezogene kreisförmige Gießformen mit einem Durchmesser 50 mm und einer Tiefe von 3 mm die Haut kontaktierende Schichten in einer Dicke von 500 µm vergossen. Die vergossenen Schichten wurden während einer Zeitspanne von 15 Sekunden mit UV-Licht bestrahlt und auf eine Temperatur von 60°C erwärmt.

Anschließend wurde auf diese Schicht ein runder plattenförmiger Sensor mit einem Durchmesser von 6 mm und einer Dicke der Sensorplatte von 1 mm aufgesetzt.

Anschließend wurde eine durchsichtige Mischung aus 60 g Propandiol, 5 g Glycidetheracrylat, 40 g Tripropylenglykoldiacrylat, 60 g Polyethylenglykol-300 und 3 g 2-Hydroxy-2-methyl-1-phenylpropan-1-on als Deckschicht in einer Dicke von 800 µm auf dem erhaltenen Gebilde vergossen und während einer Zeitspanne von 15 Sekunden bei einer Temperatur von 60°C unter Einwirkung von UV-Licht ausgehärtet.

Die dabei erhaltene Körperelektrode war an den nicht von der Sensorplatte abgedeckten Stelle durchsichtig und klebte ohne Ausübung von Reizwirkunen gut an der menschlichen Haut an.

## Patentansprüche

1. Körperelektrode mit einer an der Haut haftenden Schicht (4) und einer nichtklebenden nichtleitenden Deckschicht (5) sowie einem oder mehreren galvanisch aktiven Sensor(en) (1), der (die) an der Körperkontaktseite mit der an der Haut haftenden Schicht (4) aus einem elektrisch leitenden klebenden elastischen hydrophilen Material verbunden ist (sind), wobei die der Körperkontaktseite abgewandte Seite des Sensors bzw. der Sensoren und der an der Haut haftenden Schicht (4) mit der nichtklebenden nichtleitenden Deckschicht (5) abgedeckt sind, und die an der Haut haftende Schicht (4) und die nichtleitende Deckschicht (5) aus jeweils einem vergießbaren Polymer bestehen, welches jeweils in eine Form gegossen und anschließend ausgehärtet worden ist, wobei Größe und Ausgestaltung der Form so gewählt sind, daß diese der an der Haut haftenden Schicht entspricht.

2. Körperelektrode nach Anspruch 1, dadurch gekennzeichnet, daß das Verhältnis der Fläche der an der Haut haftenden Schicht (4) zu der Fläche des Sensors bzw. der Sensoren (1) größer als 1, insbesondere 2 bis 3, ist.

3. Körperelektrode nach Anspruch 1 oder 2, dadurch gekennzeichnet, daß die an der Haut haftende Schicht (4) ein klebrigmachendes Mittel, einen Weichmacher und/oder ein hygroskopisches Mittel enthält.

4. Körperelektrode nach einem der vorhergehenden Ansprüche, dadurch gekennzeichnet, daß die an der Haut haftende Schicht (4) und die Deckschicht (5) die selben vernetzbaren nichtleitenden Polymeren aufweisen, wobei das Polymere der Deckschicht stärker vernetzt ist.

5. Körperelektrode nach den Ansprüchen 1 bis 4, dadurch gekennzeichnet, daß die Sensoren und/oder elektrischen Leitungen aus Schichten (15,11) aus galvanisch aktiven Materialien bestehen, die auf eine Folie (13) insbesondere durch Siebdruck aufgebracht worden sind.

6. Körperelektrode nach den Ansprüchen 1 bis 5, dadurch gekennzeichnet, daß die vergossene, an der Haut haftende Schicht (4) und/oder die Deckschicht (5) durchsichtig sind und zwischen diese beiden Schichten eine mit Zeichen versehene Schicht, die ebenfalls durchsichtig sein kann, eingebracht ist.

7. Verfahren zur Herstellung einer Körperelektrode nach Anspruch 1, wobei zur Herstellung der an der Haut haftenden Schicht (4) ein elektrisch leitendes oder leitend gemachtes, klebriges elastisches hydrophiles Polymer und zur Herstellung der nichtleitenden Deckschicht (5) ein nichtklebendes nichtleitendes Polymer in vergießbarer Form eingesetzt wird, wobei nach Eingiessen einer Schicht und hinreichender Verfestigung derselben der Sensor eingelegt und die zweite Schicht aufgegossen und anschließend ausgehärtet wird, und wobei Größe und Ausgestaltung der Form so gewählt sind, daß diese der an der Haut haftenden Schicht (4) entspricht.

8. Verfahren nach Anspruch 7, dadurch gekennzeichnet, daß zum Vergießen der an der Haut haftenden Schicht (4) und der Deckschicht (5) je eine Schmelze, Lösung oder eine vernetz- beziehungsweise härtbare Vorstufe des zu vergießenden Materials verwendet wird.

9. Verfahren nach den Ansprüchen 7 oder 8, dadurch gekennzeichnet, daß in der Gießform die an der Haut haftende Schicht (4) vergossen und ausgehärtet wird, ein Sensor mit elektrischer Anschlußmöglichkeit eingelegt und anschließend die Deckschicht (5) unter Einbettung des Sensors vergossen wird.

10. Verfahren nach den Ansprüchen 7 bis 9, dadurch gekennzeichnet, daß in die zur Herstellung der Elektrode verwendete Form eine Folie mit einer darauf aufgebrachten, galvanisch aktiven Schicht eingelegt und die an der Haut haftende Schicht (4) über der galvanisch aktiven Schicht vergossen wird.

## Claims

1. Body electrode having a layer (4) which adheres to the skin and a non-adhesive, non-conductive covering layer (5) and one or more galvanically active sensor(s) (1) which is (are) bonded, on the body contact side, to the layer (4) which adheres to the skin and is made of an electroconductive adhesive elastic hydrophilic material, that side of the sensor(s) and of the layer (4) adhering to the skin, which faces away from the body contact side, being covered with the non-adhesive non-conductive covering layer (5), and the layer (4) adhering to the skin, and the non-conductive covering layer (5) each consisting of a castable polymer which has in each case been poured into a mould and then been cured, the size and shape of the mould being selected in such a way that it corresponds to the layer adhering to the skin.

2. Body electrode according to Claim 1, characterized in that the ratio of the area of the layer (4) which adheres to the skin, to the area of the sensor(s) (1) is greater than 1, preferably from 2 to 3.

3. Body electrode according to claim 1 or 2, characterized in that the layer (4) which adheres to the skin contains a tackifier, a plasticizer and/or a hygroscopic agent.

4. Body electrode according to any one of the preceding claims, characterized in that the layer (4) which adheres to the skin, and the covering layer (5) contain the same cross-linkable non-conductive polymers, the polymer of the covering layer being cross-linked more strongly.

5. Body electrode according to any of claims 1 to 4, characterized in that the sensors and/or electrical conductors consist of layers (15, 11) of galvanically active materials which have been applied to a film (13) preferably by screen-printing.

6. Body electrode according to any of claims 1 to 5, characterized in that the cast layer (4) which adheres to the skin, and/or the covering layer (5) are transparent and between these two layers there is introduced a layer which is provided with symbols and may likewise be transparent.

7. Process for fabricating a body electrode according to claim 1, wherein, to produce the layer (4) which adheres to the skin, a tacky, elastic hydrophilic polymer which is or has been made electroconductive is employed, and to produce the non-conductive covering layer (5) a non-adhesive, non-conductive polymer in a castable form is employed, the process involving, after a layer has been poured in and has sufficiently solidified, inserting the sensor and pouring on and then curing the second layer, the size and shape of the mould being selected in such a way that it corresponds to the layer (4) adhering to the skin.

8. Process according to claim 7, characterized in that the casting of the layer (4) which adheres to the skin, and of the covering layer (5) employs one melt, solution, or cross-linkable or curable precursor each of the material to be cast.

9. Process according to either of claims 7 or 8, characterized in that, in the casting mould, the layer (4) which adheres to the skin is cast and cured, a sensor which can be connected electrically is inserted, and the covering layer (5) is then cast, embedding the sensor.

10. Process according to any of claims 7 to 9, characterized in that there is inserted, in the mould used to fabricate the electrode, a film with a galvanically active layer applied thereto, and the layer (4) which adheres to the skin is cast above the galvanically active layer.

## Revendications

1. Electrode corporelle, avec une couche (4) adhérant à la peau et une couche de couverture (5) non conductrice, non adhésive, ainsi qu'un ou plusieurs capteurs (1), actifs par voie galvanique, relié(s) par la face de contact corporelle, à la couche (4) adhérant sur la peau, composée d'un matériau hydrophile, élastique, adhésif, conducteur de l'électricité, la face, opposée à cette face de contact corporelle, du capteur ou des capteurs et la couche (4) adhérant à la peau étant couverte par la couche de couverture (5) non conductrice, non adhésive, et la couche (4) adhérant à la peau et la couche de couverture (5) non conductrice étant composées chacune d'un polymère pouvant être coulé, ayant été chaque fois coulé dans un moule, puis durci, la taille et la configuration du moule étant telles que celles-ci correspondent à la taille et la configuration de la couche adhérant sur la peau.

2. Electrode corporelle selon la revendication 1, caractérisée en ce que le rapport entre la surface de la couche (4) adhérant à la peau et la surface du ou des capteurs (1) est supérieur à 1, en particulier est compris entre 2 et 3.

3. Electrode corporelle selon la revendication 1 ou 2 caractérisée en ce que la couche (4) adhérant à la peau contient un produit à action adhésive, un plastifiant et/ou un agent hygroscopique.

4. Electrode corporelle selon l'une des revendications précédentes, caractérisée en ce que la couche (4) adhérant à la peau et la couche de couverture (5) présentent les mêmes polymères, non conducteurs, réticulables, le polymère de la couche de couverture étant réticulé plus fortement.

5. Electrode corporelle selon la revendication 1 à 4 caractérisée en ce que les capteurs et/ou les lignes électriques venant des couches (15, 11) sont réalisées en des matériaux actifs galvaniquement, ayant été appliqués en particulier par sérigraphie sur une feuille (13).

6. Electrode corporelle selon les revendications 1 à 5 caractérisée en ce que la couche (4) adhérant à la peau, coulée et/ou la couche de couverture (5) sont transparentes et une couche, pourvue de signes, pouvant également être transparente, étant intégrée entre ces deux couches.

7. Procédé de fabrication d'une électrode corporelle selon la revendication 1, un polymère hydrophile, élastique, adhésif conducteur ou rendu conducteur de l'électricité, étant utilisé pour la fabrication de la couche (4) adhérant à la peau et un polymère non conducteur, non adhésif, étant utilisé sous une forme pouvant être coulée, pour la fabrication de la couche de couverture (5) non conductrice, le capteur étant inséré après coulée d'une couche et solidification suffisante de celle-ci et la deuxième couche étant coulée puis durcie, la taille et la configuration du moule étant choisies telles que le moule correspond à la couche (4) adhérant à la peau.

8. Procédé selon la revendication 7, caractérisé en ce que, pour couler la couche (4) adhérant à la peau et la couche de couverture (5), on utilise respectivement un produit en fusion, une solution, une solution ou bien un produit situé à stade préliminaire, pouvant être réticulé, respectivement durci, du matériau à couler.

9. Procédé selon les revendications 7 ou 8, caractérisé en ce que la couche (4) adhérant à la peau et coulée et durcie dans le moule de coulée, un capteur à possibilité de raccordement électrique étant inséré, puis la couche de couverture (5) étant coulée en noyant le capteur.

10. Procédé selon les revendications 7 à 9, caractérisé en ce qu'une feuille, ayant une couche active galvaniquement appliquée sur elle, est insérée dans le moule utilisé pour la fabrication de l'électrode et la couche (4) adhérant à la peau étant coulée au-dessus de la couche active galvaniquement.
